(19) Europäisches Patentamt
European Patent Office
Office européen des brevets

(11) EP 2 926 811 B1

(12) EUROPEAN PATENT SPECIFICATION

(45) Date of publication and mention of the grant of the patent:
**06.01.2021 Bulletin 2021/01**

(21) Application number: **13857684.8**

(22) Date of filing: **27.11.2013**

(51) Int Cl.:
*A61K 31/357* (2006.01) *A61K 9/00* (2006.01)
*A61P 35/00* (2006.01) *A61K 33/26* (2006.01)
*A61K 9/19* (2006.01) *A61P 43/00* (2006.01)

(86) International application number:
**PCT/CN2013/087890**

(87) International publication number:
**WO 2014/082570 (05.06.2014 Gazette 2014/23)**

# (54) ARTEMETHER-CONTAINING PHARMACEUTICAL COMPOSITION, PREPARATION, AND USE THEREOF

ARTEMETHERHALTIGE PHARMAZEUTISCHE ZUSAMMENSETZUNG, HERSTELLUNG UND VERWENDUNG

COMPOSITION PHARMACEUTIQUE COMPRENANT DE L'ARTÉMÉTHER, PRÉPARATION, ET UTILISATION ASSOCIÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.11.2012 CN 201210499289**

(43) Date of publication of application:
**07.10.2015 Bulletin 2015/41**

(73) Proprietor: **KPC Pharmaceuticals, Inc.**
**Kunming, Yunnan 650106 (CN)**

(72) Inventors:
- **QING, Chen**
  **Yunnan 650106 (CN)**
- **CHEN, Yunjian**
  **Yunnan 650106 (CN)**
- **DAI, Xiaoyang**
  **Yunnan 650106 (CN)**
- **ZHANG, Yanli**
  **Yunnan 650106 (CN)**
- **CHEN, Yajuan**
  **Yunnan 650106 (CN)**
- **LIU, Yidan**
  **Yunnan 650106 (CN)**
- **YANG, Zhaoxiang**
  **Yunnan 650106 (CN)**
- **YANG, Xujuan**
  **Yunnan 650106 (CN)**
- **ZHU, Ze**
  **Yunnan 650106 (CN)**

(74) Representative: **Potter Clarkson**
**The Belgrave Centre**
**Talbot Street**
**Nottingham NG1 5GG (GB)**

(56) References cited:
**EP-A1- 1 658 844** **CN-A- 1 520 823**
**CN-A- 1 668 293** **US-A- 5 578 637**

- **DATABASE WPI Week 200479 Thomson Scientific, London, GB; AN 2004-797033 XP002759228, & CN 1 327 842 C (SHEN H) 25 July 2007 (2007-07-25)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 926 811 B1

## Description

## TECHNICAL FIELD

The present invention relates to the field of medicine, in particular to an artemether-containing pharmaceutical composition, a preparation thereof and use thereof in the preparation of a medicament for the treatment of a tumor.

## BACKGROUND OF THE INVENTION

Artemether, having a chemical name of (3R,5αS,6R,8αS,9R,10S,12R,12αR)-decahydro-10-methoxy-3,6,9-trimethyl-3,12-epoxy-12H-pyrano[4,3-j]-1,2-benzodioxepin, a molecular formula of $C_{16}H_{26}O_{25}$ and a structural formula as the following:

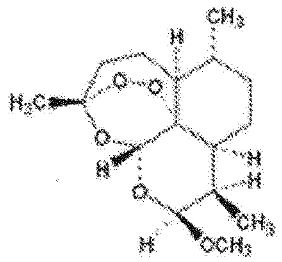

is used for various types of malaria, mostly for treatment of chloroquine-resistant falciparum malaria and for first aid of pernicious falciparum malaria.

It is acknowledged that artemisinin and derivatives thereof have an antimalarial efficacy. Studies have found that, the active *in vivo* metabolite of artemisinin and its derivatives is dihydroartemisinin, which has a high antimalarial activity and low toxicity. Many scholars conducted detailed studies on the peroxide bridge in the structure of artemisinin, and summarized the antimalarial mechanism thereof into the following two steps: artemisinin is activated to produce free radicals, and the free radicals in turn bind to the proteins of plasmodium. When the peroxide bridge is reduced and decomposed to form free radicals, the presence of a low-valence metal ion in a transition state is required. The currently acknowledged metal ion related to the generation of free radicals is ferrous ion ($Fe^{2+}$), including ferroheme and free $Fe^{2+}$. After the peroxide bridge is catalytically broken by $Fe^{2+}$, oxygen free radicals are firstly produced, and subsequently converted into more active carbon free radicals via molecular rearrangement. Both of these two kinds of free radicals destroy the cell structure and function of plasmodia; while carbon free radicals can directly alkylate macromolecules of a variety of organisms, it is thus now believed that artemisinin is more likely to achieve its activity through carbon free radicals. Document CN1327842 discloses a combination of arthemeter and metal ions for use in the treatment of cancer.

Researches in recent years show that anti-malarial drug, artemisinin, and its derivatives exhibit a significant antitumor activity, thus studies on their pharmacological effects have received wide attention. Artemether is one of the main derivatives of artemisinin, and exhibits a better antimalarial activity than artemisinin. Artemisinin-based drugs can not only selectively inhibit and/or kill various tumor cells, but also have very little side effects, and even do not result in drug tolerance. The antitumor activity of artemisinin-based drugs have been confirmed in multiple aspects, and the main action mechanism thereof is as the follows:

1. Cell damage mediated by iron ion: Malignant tumor cells have higher Fe content than normal cells. Artemisinin-based drugs can form endoperoxide bridge by mediation of iron ion, and produce reactive oxygen species (ROS) and carbon-centered free radicals, wherein the latter will cause large molecular damage and death of cell.

2. Cell cycle arrest: Artesunate can significantly reduce cell cycle of Kaposi's sarcoma, and dihydroartemisinin may arrest human breast cancer MCF-7 cell in the G0+G1 phase.

3. Induce apoptosis: artesunate can induce apoptosis of Kaposi's sarcoma in a dosage-dependent manner, but cannot induce apoptosis of normal cells. Artemisinin can induce apoptosis of human erythroleukemia K562 cells by reducing transmembrane potential.

4. Anti-angiogenic effect: Artemisinin-based drugs can prevent the neovascularization of tumor by inhibiting the expression of VEGF and KDR/flk-1.

5. Artemisinin-based drugs can regulate the expression of tumor-associated genes, and thereby achieve antitumor activity.

6. Others: For example, artesunate has the ability against multidrug resistance. Artemisinin can inhibit the synthesis of nuclear factor NF-κB in astrocytoma T67 cell. Furthermore, artemether may induce damage and swell of mitochondria, fracture, reduction and disappearance of crista, as well as inhibit the activity of complexes I and IV of

mitochondrial respiratory chain, and thereby result in cell death.

## SUMMARY OF THE INVENTION

The invention is as defined in the claims.

The technical problem to be solved by the present invention is to provide a pharmaceutical composition for treatment of tumor, containing artemether together with an iron agent.

The weight ratio of artemether to the iron agent is 1:0.05 to 1:2.

Preferably, the weight ratio of artemether or dihydroartemisinin to the iron agent is 1:0.1 to 1:1.

The tumor is liver cancer or gastric cancer.

The present invention further provides a pharmaceutical preparation, consisting of the pharmaceutical composition and a pharmaceutically acceptable excipient. Preferably, the pharmaceutical preparation is an injection or lyophilized powder for injection.

The present invention further provides use of a composition comprising artemether together with an iron agent in the preparation of a medicament for treatment of a tumor.

The weight ratio of artemether or dihydroartemisinin to the iron agent in the composition is 1:0.05 to 1:2.

Preferably, the weight ratio of artemether or dihydroartemisinin to the iron agent in the composition is 1:0.1 to 1:1.

In the present invention, the *in vitro* anti-tumor activity of artemether and its lyophilized powder were determined by a modified MTT method with seven human tumor cell lines as models, compared with artemisinin, dihydroartemisinin and artesunate. The results showed that, neither artemether nor its lyophilized powder has apparent *in vitro* inhibitory effect on the seven human tumor cell lines: human leukemia cell lines (HL-60, K562), human lung cancer cell line (A549), human colon cancer cell line (HT-29) and human prostate cancer cell line (PC-3), human gastric cancer cell line (MKN-28), human laryngeal cancer cell line (Hep2). Dihydroartemisinin has relatively apparent inhibitory effect on five of the tumor cell lines: HL-60, K562, MKN-28, HT-29 and Hep2, especially being sensitive to MKN-28 cell, with an $IC_{50}$ of 16.22 $\mu$M. Lyophilized powder of artemether is a water-soluble preparation which is different from other derivatives in the *in vitro* studies, thus it is possible to perform anti-tumor assessment through intravenous injection. Therefore, although experimental results showed that neither artemether nor its lyophilized powder has apparent *in vitro* cytotoxicity, it may be metabolically converted into dihydroartemisinin in an organism in consideration of the retaining of the pharmacophore peroxide bridge of artemisinin. As such, the present invention performed the *in vivo* anti-tumor study using the lyophilized powder of artemether.

The *in vivo* experimental results showed that, upon administering lyophilized powder of artemether by intraperitoneal injection at a dosage of 5, 10, 20 mg/kg once a day for continuous 10 days, the tumor inhibition rates of mouse sarcoma S180 were 29.06%, 44.96% and 54.93%, respectively, showing remarkable tumor inhibitory effect and good dosage dependence; and each dosage group has a significant difference by statistical analysis as compared with a negative control group. When comparing administrations by intraperitoneal injection and intravenous injection at a dosage of 10 mg/kg respectively, it was found that, the effect of intravenous administration is superior to that of intraperitoneal administration, with the tumor inhibitory rates being 44.96% (ip) and 51.48% (iv), respectively.

Based on the above experiments, according to the currently acknowledged mechanism of action of oxygen free radicals, mouse liver cancer H22 was used as a model in the present invention, and the dosage and study protocol was adjusted. Different influencing factors were designed and the dosage was adjusted in *in vivo* experiments, so as to verify the mechanism of its anti-tumor action. The results showed that, administration of the lyophilized powder of artemether by intravenous injection at a dosage of 3, 10, 30 mg/kg, once a day for continuous 10 days, showing apparent tumor inhibitory effect and good dose dependence, and the tumor inhibitory rates thereof were 38.98%, 54.80% and 73.07%, respectively. On the basis of the 10 mg/kg dosage of the lyophilized powder of artemether, we separately designed an iron addition group, Vit C addition group and deferoxamine (DFO) addition group, i.e.: 10 mg/kg (iv), 10 mg/kg+$FeSO_4$ 3 mg/kg (ig), 10 mg/kg+Vit C 50 mg/kg (im) and 10 mg/kg+DFO 30 mg/kg (im). The purpose is to investigate the role of $Fe^{2+}$ in the anti-tumor effect of artemether. Compound ferrous sulfate and folic acid provides ferrous ions; deferoxamine (DFO) is an iron chelator which is capable of forming a chelate with *in vivo* ferrous ions $Fe^{2+}$ to remove the *in vivo* ferrous ions; and the Vit C group is used against the *in vivo* oxidation reaction of a mouse. The tumor inhibitory rates of the groups were 54.80%, 61.15%, 59.60% and 39.92%, respectively. Compared with administration of the lyophilized powder of artemether alone, the tumor inhibitory rate in the iron addition group increased, while that in the DFO group decreased, which suggested that the mechanism of the anti-tumor effect of the lyophilized powder of artemether is related to $Fe^{2+}$ and closely related to $Fe^{2+}$ content. In consideration of the short experiment period, endogenous $Fe^{2+}$ inside the animal body did not have apparent change in short time and could not sufficiently display the inhibitory effect on tumor growth. Owing that a nude-mouse tumor xenograft human carcinomas model retains biological properties of human tumor to a large extent, and grows more slowly than the tumor xenograft from an animal, thus differs greatly in biological properties from animal-derived tumor and retains to a large extent the biological properties of human tumor. Therefore, we conducted experiments using a nude-mouse tumor xenograft human carcinomas model, and further

objectively assess the efficacy and mechanism of action thereof.

The *in vitro* cell screening results show that human gastric cancer line MKN-45 is the most sensitive to dihydroartemisinin, and thus a nude-mouse subcutaneous transplanted human gastric cancer MKN-45 tumor model was selected to conduct experiments. According to the results of H22 mouse model, the dosage of Vit C was adjusted, which was increased from original 150 mg/kg to 200 mg/kg. The lyophilized powder of artemether was administered via intravenous injection at a dosage of 3, 10g, 30 mg/kg once a day for five days a week. On day 19 upon administration, T/C (%) of tumor tissues was 50.68%, 48.43%, and 47.32%, respectively, showing the inhibitory effect on tumor growth with a certain dosage relationship. Compared with the negative control, the dosages 10 and 30 mg/kg have significant differences by statistical analysis with $P < 0.05$. The positive control group 5-Fu has a T/C (%) of 37.72% at a dosage of 10 mg/kg, which has a significant difference by statistical analysis with $P < 0.05$. It can be deemed that the model was established under the conditions, and the experimental results are credible. On day 19 upon administration of the lyophilized powder of artemether (10 mg/kg), lyophilized powder of artemether (10 mg/kg) + $FeSO_4$ (3 mg/kg), lyophilized powder of artemether (10 mg/kg) + Vit C (200 mg/kg), lyophilized powder of artemether (10 mg/kg) + DFO (30 mg/kg), T/C (%) of the tumor tissue was 48.43%, 48.38%, 96.36% and 91.95%, respectively. Compared with administration of the lyophilized powder of artemether at 10 mg/kg alone, T/C (%) of the $FeSO_4$ group has no apparent change, suggesting that under this experiment condition, exogenous iron has no apparent influence on the inhibition effect of artemether on tumor growth. However, as the time after administration goes, $FeSO_4$ gradually exhibits enhancement of the inhibition effect of artemether on tumor growth. It is assumed that at the early stage of experiments, endogenous $Fe^{2+}$ is capable of meeting the demand of artemether to exert the anti-tumor effect, but with time on, the endogenous $Fe^{2+}$ is gradually consumed, and exogenous iron gradually plays a role in the enhancement of the anti-tumor strength of the artemether. T/C (%) of the DFO group dramatically rises up, suggesting that under this experiment condition, exhaustion of the endogenous $Fe^{2+}$ inside an animal is capable of counteracting the anti-tumor effect of artemether; T/C (%) of the Vit C group dramatically rises up, suggesting that under this experiment condition, elimination of ROS inside an animal is capable of preventing artemether from exerting the anti-tumor effect thereof.

## BRIEF DESCRIPTION OF THE DRAWINGS

Figure 1 shows the inhibitory effect of artemether on the growth of mouse sarcoma S180, wherein H represents lyophilized powder of artemether;

Figure 2 shows the tumor inhibitory effect of a lyophilized powder preparation of artemether on the growth of mouse sarcoma S180;

Figure 3 shows the inhibitory effect of lyophilized powder of artemether on the growth of mouse liver cancer H22, wherein H represents lyophilized powder of artemether;

Figure 4 shows the inhibitory effect of lyophilized powder of artemether on the growth of mouse liver cancer H22, wherein H represents lyophilized powder of artemether;

Figure 5 shows the inhibitory effect of lyophilized powder of artemether on the growth of mouse H22 liver cancer;

Figure 6 shows the inhibitory effect of artemether on the growth of nude-mouse transplanted tumor of human gastric cancer MKN-45, wherein H represents lyophilized powder of artemether;

Figure 7 shows the inhibitory effect of artemether on the growth of nude-mouse transplanted tumor of human gastric cancer MKN-45.

## DETAILED DESCRIPTION OF THE INVENTION

The present invention discloses a pharmaceutical composition containing artemether, a preparation and use thereof in the preparation of a medicament for treatment of a tumor. The use of the present invention has been described by examples.

The present invention will be further illustrated in detail in combination with examples in order to render those skilled in the art to understand the present invention better.

EXAMPLE 1: Influence of lyophilized powder preparation of artemether on the growth of human tumor cells (compared with other derivatives of artemisinin)

(I) Materials and Methods

(1) Test samples and formulation thereof

1. Test samples

A preparation of lyophilized powder of artemether, artemether drug substance, artemisinin, dihydroartemisinin, artesunate, provided by Institute of Kunming Pharmaceutical Corp..

(2) Samples and formulation thereof

① Formulation of test samples

Five test concentrations 2, 10, 50, 250 and 500 μM were set for each of five derivatives of artemisinin, artemether drug substance, artemisinin, dihydroartemisinin, artesunate were dissolved with dimethyl sulfoxide (DMSO), while the lyophilized powder of artemether was dissolved with normal saline (N.S.), followed by addition of a certain amount of RPMI 1640 complete culture solution to formulate a high-concentration solution, and each of the low-concentration solutions was formulated by proportionate dilution from the high-concentration solution.

② Formulation of solvent controls

The concentrations of DMSO contained in the solutions at the two relatively high concentrations 500 and 250 μM for the artemether drug substance, artemisinin, dihydroartemisinin, artesunate, used as solvent controls, were formulated with PRMI 1460 culture solution, at which time the test concentrations of DMSO were 1% and 0.5%. The concentrations of the excipients contained in the solutions at the two relatively high concentrations 500 and 250 μM for the preparation of lyophilized powder of artemether were used as solvent controls.

③ Formulation of positive controls

The positive control was an anti-cancer drug adriamycin (ADM), which was set at three test concentrations (final concentrations) of 0.1, 1 and 10 μg/ml. To ADM stock solution (1 mg/ml) a certain amount of PRMI 1640 complete culture solution was added, to formulate a high-concentration solution, and each of the low-concentration solutions were formulated by proportionate dilutions from the high-concentration solution.

2. Cell lines

HL-60: (human promyelocytic leukemia cell line); K562 (human chronic myelocytic leukemia cell line); HT-29 (human colon cancer cell line); PC-3 (human prostate cancer cell line); A549 (human non-small cell lung cancer cell line); MKN-28 (human gastric cancer cell line); Hep2 (human laryngeal cancer cell line), all of which were purchased from Cell Bank of Shanghai Institutes for Biological Sciences, the Chinese Academy of Sciences.

3. Detection method (MTT method)

Influence of the five test samples on the proliferation of 7 human tumor cell lines was determined via a modified MTT method.

Cells in logarithmic growth phase were taken, adjusted to a certain concentration and then added into a 96 well culture plate. After culture of adherent cells for a period until adherence, the test samples were added; and for suspension cells, the test samples were added after inoculation. 5 test concentrations 2, 10, 50, 250 and 500 μM were set, with 3 parallel wells for each concentration, 10 μl/well; the solvent controls were 0.5% and 1% of isovolumetric DMSO, and the positive controls was ADM at 3 concentrations 0.1, 1 and 10 μg/ml. The samples were loaded followed by culture for a period of time in an incubator at 37°C, 5% $CO_2$, followed by addition of MTT (5 mg/ml) at 20 μl/well, and continued to be cultured for a period of time and added with a three-combined solution [10% SDS - 5% isobutanol - 0.012 mol/L HCl (W/V/V)]. After dissolving in uniformity, it was continued to be placed in the incubator at 37°C, 5% $CO_2$ for a period of time, and the OD value of each well was determined under a wavelength of 570 nm with a microplate reader, according to which the results were calculated.

3. Result calculation and statistical analysis

The cellular proliferation inhibition rate was calculated in accordance with the following equation:

$$\text{Inhibition rate (\%)} = (\text{OD value}_{\text{control well}} - \text{OD value}_{\text{sample well}}) / \text{OD value}_{\text{control well}} \times 100\%$$

When the inhibition rate of DMSO on cells was more than 15%, calculation was carried out using the following equation:

$$\text{Inhibition rate (\%)} = (\text{OD value}_{\text{solvent group}} - \text{OD value}_{\text{administered well}}) / \text{OD value}_{\text{solvent group}} \times 100\%$$

The inhibition rate was calculated by using Microsoft Excel 2003, and median inhibitory concentration $IC_{50}$ was calculated with a LOGIT method, which was used as an indicator for judging the inhibitory capability.

(II) Results and analysis

1. Inhibitory effect of 5 test samples on the growth of 7 human tumor cell lines is shown in Tables 1-5.

Table 1. $IC_{50}$ ($\mu$M) of five test samples on the growth of K562 cells

| sample batch | test duration (h) | artemisinin | dihydroartemisinin | artemether drug substance | artesunate | lyophilized powder of artemether | ADM (positive) |
|---|---|---|---|---|---|---|---|
| Batch 1 | 24 h | > 1000 | > 1000 | > 1000 | > 1000 | 551.04 | 7.09 |
| | 48 h | > 1000 | 996.6 | > 1000 | 322.47 | 150.21 | 1.90 |
| | 72 h | > 1000 | 491.7 | > 1000 | 39.26 | 48.26 | 0.17 |
| Batch 2 | 24 h | > 1000 | 144.06 | > 1000 | > 1000 | > 1000 | 5.76 |
| | 48 h | > 1000 | 27.97 | > 1000 | > 1000 | 473.45 | 0.24 |
| | 72 h | 517.69 | 44.82 | 892.61 | > 1000 | 209.31 | $9.93\times10^{-3}$ |
| Batch 3 | 24 h | > 1000 | 146.34 | 345.21 | 493.61 | 450.17 | 2.59 |
| | 48 h | 991.25 | 80.72 | 331.11 | 140.82 | 543.641 | 1.69 |
| | 72 h | 448.96 | 60.54 | 137.27 | 90.13 | 292.35 | 0.74 |

Table 2. $IC_{50}$ ($\mu$M) of five test samples on the growth of HL-60 cells

| sample batch | test duration (h) | artemisinin | dihydroartemisinin | artemether drug substance | artesunate | lyophilized powder of artemether | ADM (positive) |
|---|---|---|---|---|---|---|---|
| Batch 1 | 24 h | > 1000 | > 1000 | > 1000 | > 1000 | 982.55 | 9.24 |
| | 48 h | > 1000 | > 1000 | > 1000 | > 1000 | 521.16 | 1.72 |
| | 72 h | > 1000 | > 1000 | > 1000 | > 1000 | 149.25 | 0.78 |
| Batch 2 | 24 h | 749.92 | 74.35 | 658.09 | 269.03 | 248.31 | 4.19 |
| | 48 h | 330.74 | 16.26 | 77.17 | 39.51 | 124.67 | 0.16 |
| | 72 h | 577.06 | 115.59 | 248.31 | 145.40 | 426.07 | 0.57 |

Table 3. $IC_{50}$ (μM) of five test samples on the growth of A549 cells

| sample batch | test duration (h) | artemisinin | dihydroartemisinin | artemether drug substance | artesunate | lyophilized powder of artemether | ADM (positive) |
|---|---|---|---|---|---|---|---|
| Batch 1 | 24 h | > 1000 | > 1000 | > 1000 | > 1000 | > 1000 | 38.76 |
| | 48 h | > 1000 | > 1000 | > 1000 | > 1000 | > 1000 | 10.72 |
| | 72 h | > 1000 | > 1000 | > 1000 | > 1000 | > 1000 | 0.76 |
| Batch 2 | 24 h | 420.92 | 934.91 | 640.55 | > 1000 | > 1000 | > 100 |
| | 48 h | > 1000 | 221.01 | > 1000 | 272.44 | > 1000 | 0.62 |
| | 72 h | > 1000 | 192.36 | 831.51 | 225.05 | > 1000 | 0.19 |

Table 4. $IC_{50}$ (μM) of five test samples on the growth of HT-29 cells

| sample batch | test duration (h) | artemisinin | dihydroartemisinin | artemether drug substance | artesunate | lyophilized powder of artemether | ADM (positive) |
|---|---|---|---|---|---|---|---|
| Batch 1 | 24 h | > 1000 | 288.02 | > 1000 | > 1000 | > 1000 | 2.28 |
| | 48 h | > 1000 | 336.88 | > 1000 | > 1000 | 836.76 | 0.28 |
| | 72 h | > 1000 | 160.37 | 317.71 | > 1000 | 363.50 | $5.7 \times 10^{-2}$ |
| Batch 2 | 24 h | > 1000 | 280.34 | > 1000 | 323.21 | > 1000 | 3.81 |
| | 48 h | > 1000 | 432.93 | > 1000 | > 1000 | 756.17 | 0.59 |
| | 72 h | > 1000 | 257.50 | 579.11 | 438.21 | 568.17 | 0.11 |

Table 5. $IC_{50}$ (μM) of five test samples on the growth of PC-3 cells

| sample batch | test duration (h) | artemisinin | dihydroartemisinin | artemether drug substance | artesunate | lyophilized powder of artemether | ADM (positive) |
|---|---|---|---|---|---|---|---|
| Batch 1 | 24 h | > 1000 | 659.58 | > 1000 | > 1000 | > 1000 | 33.02 |
| | 48 h | > 1000 | > 1000 | > 1000 | > 1000 | > 1000 | 7.60 |
| | 72 h | > 1000 | 230.34 | > 1000 | > 1000 | 379.66 | 3.16 |
| Batch 2 | 24 h | > 1000 | > 1000 | > 1000 | > 1000 | > 1000 | 47.83 |
| | 48 h | > 1000 | > 1000 | > 1000 | > 1000 | > 1000 | 9.62 |
| | 72 h | > 1000 | > 1000 | > 1000 | > 1000 | > 1000 | 3.74 |

According to the results of preliminary screening, further experiments were adjusted such that more sensitive cell lines were screened out. As the lyophilized powder of artemether contains a large amount of excipients and has relatively high swelling coefficient with large error, only artemether drug substance was selected for secondary screening, and the results are shown in Tables 6-8.

Table 6. $IC_{50}$ (μM) of four test samples on the growth of HT-29 cells (72 h)

| sample | test batch | artemisinin | dihydroartemisinin | artemether drug substance | artesunate | ADM positive |
|---|---|---|---|---|---|---|
| HT-29 | Batch 1 | 2349.92 | 236.40 | 213.96 | 328.41 | 0.11 |
| | Batch 2 | 772.95 | 172.80 | 262.66 | 306.08 | 0.19 |
| | Batch 3 | 436.91 | 148.49 | 245.23 | 199.72 | 0.12 |
| | average | 544.75 | 143.00 | 254.83 | 225.27 | 0.11 |

Table 7. $IC_{50}$ (μM) of four test samples on the growth of MKN-28 cells (72 h)

| sample test batch | | artemisinin | dihydroartemisinin | artemether drug substance | artesunate | ADM positive |
|---|---|---|---|---|---|---|
| MKN-28 | Batch 1 | 587.76 | 15.10 | 27895.65 | 35.30 | 0.52 |
| | Batch 2 | 1438.90 | 17.07 | 336.32 | 28.10 | 0.53 |
| | Batch 3 | 1612.26 | 16.50 | 519.55 | 21.40 | 0.47 |
| | Average | 970.36 | 16.22 | 402.40 | 27.92 | 0.51 |

Table 8. $IC_{50}$ (μM) of found test samples on the growth of Hep2 cells (72 h)

| sample test batch | | artemisinin | dihydroartemisinin | artemether drug substance | artesunate | ADM positive |
|---|---|---|---|---|---|---|
| Hep2 | Batch 1 | 350.26 | 113.72 | 412.44 | 119.21 | 0.41 |
| | Batch 2 | 271.29 | 88.17 | > 1000 | 93.22 | 0.26 |
| | Batch 3 | > 1000 | 125.54 | > 1000 | 212.06 | 1.00 |
| | average | 951.78 | 91.96 | > 1000 | 149.46 | 0.51 |

The above results show that artemisinin, artemether and lyophilized powder thereof have no apparent inhibitory effect on the 7 human tumor cell lines *in vitro,* artesunate only shows inhibitory effect on HL-60, K562, MKN-28; while dihydroartemisinin has relatively apparent inhibitory effect on five tumor cell lines HL-60, K562, MKN-28, HT-29, Hep2, especially being sensitive to MKN-28 cells with an $IC_{50}$ of 16.22 μM.

EXAMPLE 2: Influence of a lyophilized powder preparation of artemether on the growth of mouse transplanted tumor

(I) Influence on the growth of mouse transplanted sarcoma S180

1. Materials and methods

(1) Test sample

A lyophilized powder preparation of artemether, provided by Institute of Kunming Pharmaceutical Corp..

(2) Formulation of the lyophilized powder preparation of artemether (the test dosage is the amount of artemether drug substance contained therein, the same below.)

A certain amount of lyophilized powder of artemether was weighed and dissolved with normal saline (N.S), and formulated into solutions with corresponding concentrations based on an administration volume of 0.2 ml/10 g body weight and a high dosage of 20 mg/kg, and the middle and low dosage were formulated by proportionate dilution with an N.S solution from the high-dosage solution.

(3) Experimental methods

① Establishment of mouse S180 models

By reference to the literature method, ascites-type S180 cells in the logarithm growth phase were taken, adjusted to a certain concentration and then subcutaneously inoculated into the right-side axilla of mice, which after 24 h were grouped randomly for administration.

② Experimental scheme

This experiment is a pre-experiment, and intended to investigate whether the lyophilized powder of artemether had *in vivo* anti-tumor effect.

Healthy female ICR mice were selected, and randomly divided into the following groups 24 hours after inoculation: negative control group (N.S); low dosage (5 mg/kg), middle dosage (10 mg/kg) and high dosage (20 mg/kg) group of lyophilized powder of artemether administered by intraperitoneal injection (ip); middle dosage (10 mg/kg) group of

lyophilized powder of artemether with intravenous administration (iv), in order to compare differences between different administration routes. The administration was performed once a day for continuous 10 days. The mice were sacrificed by cervical dislocation 24 hours after the last administration, and the tumor tissues thereof were stripped, weighed, and the tumor inhibitory rate was calculated according to the tumor weight.

③ Result calculation and analysis

The inhibitory rate of tumor growth (tumor inhibitory rate) was calculated according to the following equation.

$$\text{Tumor inhibitory rate} = (\text{average tumor weight}_{\text{control group}} - \text{average tumor weight}_{\text{administration group}})/\text{average tumor weight}_{\text{control group}} \times 100\%$$

$$\text{Thymus (spleen) index} = \text{average weight (mg)}_{\text{thymus (spleen)}}/\text{average body weight of a mouse after tumor removal (10 g)}$$

The average tumor weight of the mice in each group was expressed as x±SD, and one-way analysis of variance was performed by using SPSS 16.0 statistical software.

④ Quality control and evaluation criterion

The average tumor weight of the mice in the negative control group which was less than 1 g or the tumor weight of 20% of the mice which was less than 400 mg represented poor tumor growth. During the experiment, death of the mice in the administration group exceeding 20% or the average body weight (after tumor removal) loss (self control) exceeding 15%, represented a toxicity reaction of the medicament, for which a new trial should be performed at a reduced dosage. The inhibitory rate of tumor growth (%) < 40% means ineffectiveness, and the inhibitory rate of tumor growth (%) ≥ 40% with *P* < 0.05 by statistical treatment means efficient.

2. Results

(1) The results of the inhibitory effect of a lyophilized powder preparation of artemether on the growth of mouse sarcoma S180 tumor are shown in Table 9 and Figures 1-2.

Table 9. Inhibitory effect of a lyophilized powder preparation of artemether on the tumor growth of mouse sarcoma S180 (x±SD, n=8)

| *group* | *dosage (mg/kg)* | *administration Route* | *number of animals* | | *average tumor weight (g) (X±SD)* | tumor inhibitory rate (%) |
|---|---|---|---|---|---|---|
| | | | before experiment | after experiment | | |
| negative control (N.S) | 0.2 ml/ 10 g | ip | 9 | 9 | 2.43±0.54 | -- |
| lyophilized powder of artemether | 5 | ip | 8 | 8 | 1.72±0.76* | 29.06% |
| | 10 | ip | 8 | 8 | 1.34±0.57** | 44.96% |
| | 10 | iv | 8 | 8 | 1.18±0.61** | 51.48% |
| | 20 | ip | 8 | 8 | 1.09±0.52** | 54.93% |

Note: compared with the negative control group: ** *P* < 0.01, * *P* < 0.05
ip: administered by intraperitoneal injection; iv: administered by intravenous injection

The above results show that, after administration of the lyophilized powder preparation of artemether via intraperitoneal injection for 10 days, the tumor of the mice in the administration group has reduced tumor size and tumor weight compared with the negative control group, in which the inhibitory rates on S180 growth at the dosages 10 and 20 mg/kg are 44.96% and 54.93% respectively; while for the dosage 10 mg/kg, the inhibitory rate was increased from

44.96% (for intraperitoneal injection) to 51.48% (for intravenous injection), showing that the effect of administration by intravenous injection is superior to that of administration by intraperitoneal injection at an equivalent dosage. Each dosage group has a significant difference compared with the negative control group by statistical analysis, $P < 0.01$.

(II) Influence on the growth of transplanted liver cancer H22 in mice and verification of the mechanism of action thereof

1. Materials and methods

(1) Test sample

A lyophilized powder preparation of artemether, provided by Institute of Kunming Pharmaceutical Corp..

(2) Formulation of test samples

① Formulation of the lyophilized powder preparation of artemether

A certain amount of lyophilized powder H of artemether was weighed and dissolved with normal saline (N.S), and formulated into solutions with corresponding concentrations to a high dosage 30 mg/kg based on administration volume of 0.15 ml/10 g body weight, and the middle and low dosage (3, 10 mg/kg) were formulated by proportionate dilution with a N.S solution from the high-dosage concentration.

② Formulation of a positive control

The positive control cyclophosphamide (CTX) injection at a dosage 10 mg/kg was used at an administration volume of 0.15 ml/10 g.

It was diluted to desired concentrations with N.S.

(3) Experiment methods

① Establishment of mouse H22 models

By reference to the literature method, ascites-type H22 cells in the logarithm growth phase were taken, adjusted to a certain concentration and then subcutaneously inoculated into the right-side axilla of mice, which after 24 h were grouped randomly for administration.

② Experimental scheme

It is currently believed that the antimalarial mechanism of an arteannuinoid compound is that: ferrous ions ($Fe^{2+}$) mediate the arteannuinoid compound to produce reactive oxygen species (ROS), which may in turn oxidize protein molecules and structure of biomembranes in the interior of an insect body, thus causing the death of the insect body. We assume that ferrous ions ($Fe^{2+}$) and reactive oxygen species (ROS) would also play a role in the anti-tumor effect. Therefore, in combination with the results of the pre-experiment, the following experimental factors were added in this experimental scheme: 1. addition of iron (to increase the $Fe^{2+}$ content in the mice); 2. addition of deferoxamine (DFO) (to reduce the $Fe^{2+}$ content in the mice); 3. addition of Vit C (to eliminate reactive oxygen species (ROS)). Groups were divided experimentally as follows:

Negative control group: normal saline (NS), 0.15 ml/10 g of body weight, iv;

Positive control group: CTX 10 mg/kg, 0.15 ml/10 g, iv;

Test group (lyophilized powder of artemether)

Low dosage group: lyophilized powder of artemether 3 mg/kg, 0.15 ml/10 g, once a day, iv;

Middle dosage group: lyophilized powder of artemether 10 mg/kg, 0.15 ml/10 g, once a day, iv;

High dosage group: lyophilized powder of artemether 30 mg/kg, 0.15 ml/10 g, once a day, iv;

Middle dosage + iron group: lyophilized powder of artemether 10 mg/kg, 0.15 ml/10 g (iv), once a day + $FeSO_4$ 3 mg/kg, 0.2 ml/10 g, intragastric administration (ig), once a day;

Middle dosage + deferoxamine group: lyophilized powder of artemether 10 mg/kg, 0.15 ml/10 g (iv), once a day + deferoxamine (DFO) 30 mg/kg, 0.1 ml/10 g, administered by intramuscular injection (im), once a day;

Middle dosage + Vit C group: lyophilized powder of artemether 10 mg/kg, 0.15 ml/10 g (iv), once a day + Vit C 150 mg/kg, 0. 1 ml/10 g (im), once a day.

Healthy female ICR mice were selected, and administered 24 hours after inoculation according to the above experimental scheme for continuous 10 days. The mice were sacrificed by cervical dislocation 24 hours after the last administration, and the tumor tissues thereof were stripped, weighed, and the tumor inhibitory rate was calculated according to the tumor weight.

③ Result calculation and analysis

The inhibitory rate of tumor growth (tumor inhibitory rate) was calculated according to the following equation.

$$\text{Tumor inhibitory rate} = (\text{average tumor weight}_{\text{control group}} - \text{average tumor weight}_{\text{administration group}})/\text{average tumor weight}_{\text{control group}} \times 100\%$$

The average tumor weight of the mice in each group was expressed as x±SD, and one-way analysis of variance was performed by using SPSS 16.0 statistical software,

④ Quality control and evaluation criterion

Evaluation was performed as above.

2. Results

(1) The inhibitory effect of the lyophilized powder preparation of artemether on the growth of the mouse liver cancer H22 is shown in Table 10 and Figures 3-4.

Table 10. Inhibitory effect of lyophilized powder preparation of artemether on the tumor growth of the mouse liver cancer H22 (x±SD, n=10)

| *group* | *dosage (mg/kg)* | *administration route* | *number of animals* | | *average tumor weight (g) (X±SD)* | tumor inhibitory rate (%) |
|---|---|---|---|---|---|---|
| | | | before experiment | after experiment | | |
| negative control (N.S) | 0.2 ml/10 g | *iv* | 10 | 10 | 1.33±0.63 | -- |
| lyophilized powder of artemether | 3 | *iv* | 10 | 10 | 0.81±0.58 | 38.98% |
| | 10 | *iv* | 10 | 10 | 0.60±0.42 | 54.80% |
| | 10+$FeSO_4$ 3 mg/kg | *iv+ig* | 10 | 9 | 0.52±0.30 | 61.15% |
| | 10+DFO30 mg/kg | *iv+im* | 10 | 10 | 0.80±0.53 | 39.92% |
| | 10+Vit C 150 mg/kg | *iv+im* | 10 | 10 | 0.54±0.36 | 59.60% |
| | 30 | *iv* | 10 | 10 | 0.36±0.22 | 73.07% |
| | 10 | *iv* | 10 | 10 | 0.81±0.63 | 38.98% |

Note: *ip:* administered by intraperitoneal injection; *iv:* administered by intravenous injection; *ig:* intragastric administration; *im:* administered by intramuscular injection

The above results show that, after administration of the lyophilized powder of artemether via iv administration for 10 days, the tumor of the mice has reduced tumor size and tumor weight compared with the negative control group, in which the inhibitory rates on H22 growth with administration by intravenous injection of 10 mg/ kg and 30 mg/kg of the lyophilized powder of artemether were 54.80% and 73.07% respectively; while for the dosage 10 mg/kg, the inhibitory rate on H22 growth was increased from original 54.80% to 61.15% (in combination with iron); and when the lyophilized powder of artemether 10 mg/kg is used in combination with deferoxamine (DFO), the inhibitory rate on H22 growth was reduced from original 54.80% to 39.92%.

EXAMPLE 3: Influence of a lyophilized powder preparation of artemether on the growth of nude-mouse transplanted tumor of human gastric cancer

(I) Materials and methods

(1) Test samples and formulation thereof

1. Test samples

Lyophilized powder of artemether, provided by Institute of Kunming Pharmaceutical Corp..

(2) Formulation of test samples

A certain amount of lyophilized powder of artemether was weighed and dissolved with normal saline (N.S), and formulated into solutions with corresponding concentrations to a high dosage 30 mg/kg based on administration volume of 0.15 ml/10 g body weight, and the middle and low dosage (3, 10 mg/kg) were formulated by proportionate dilution with a N.S solution from the high-dosage concentration.

(3) Formulation of a positive control

A stock solution of 5-fluorouracil (5-Fu) was taken and diluted to desired concentrations with N.S according to an administration volume of 0.15 mg/20 g, at a dosage of 10 mg/kg.

2. Experiment methods

① Establishment of a nude-mouse tumor xenograft model of human gastric cancer

According to the *in vitro* results, a human gastric cancer cell line which was more sensitive to an arteannuinoid derivative was selected as the tumor type to be tested in the *in vivo* anti-tumor study. By reference to the literature method, a tumor tissue which grew well in a nude mouse body was taken, cut into pieces of tissues with a certain volume and subcutaneously inoculated into the right-side axilla of nude mice. The nude mice in which the tumor had no growth, very slow or rapid growth were removed several days after inoculation, and nude mice which had formed a proper tumor volume (TV) were selected to be randomly divided into groups, allowing the average value of tumor volume for each group to be close to each other.

(2) Experimental scheme

Negative control group: N.S., 0.15 ml/10 g body weight, iv;
Positive control group: 5-Fu 10 mg/kg, 0.15 ml/20 g, iv;
Test group (lyophilized powder of artemether)
Low dosage group: lyophilized powder of artemether 3 mg/kg, 0.15 ml/10 g, once a day, iv;
Middle dosage group: lyophilized powder of artemether 10 mg/kg, 0.15 ml/10 g, once a day, iv;
High dosage group: lyophilized powder of artemether 30 mg/kg, 0.15 ml/10 g, once a day, iv;
Middle dosage + iron group: lyophilized powder of artemether 10 mg/kg, 0.15 ml/10 g (iv), once a day + $FeSO_4$ 3 mg/kg, 0.2 ml/10 g (ig), once a day;
Middle dosage + deferoxamine group: lyophilized powder of artemether 10 mg/kg, 0.15 ml/10 g (iv), once a day + deferoxamine (DFO) 30 mg/kg, 0.1 ml/10 g (im), once a day;
Middle dosage + Vit C group: lyophilized powder of artemether 10 mg/kg, 0.15 ml/10 g (iv), once a day + Vit C 200 mg/kg, 0.1 ml/10 g (im), once a day.
Groups were divided according to the above experimental scheme, and administered for 5 days a week, in

continuous 4 weeks. The tumor volume (TV) was measured 3 times each week, and calculated for the relative tumor volume (RTV) and relative tumor proliferation rate (T/C %). The animals were sacrificed by cervical dislocation 3 hours after the last administration, and an intact tumor tissue were taken, weighed and the tumor inhibitory rate was calculated.

(3) Result calculation and analysis

① TV, RTV and T/C (%) were calculated in accordance with the following equation.

TV = 1/2 × a × $b^2$, in which a and b represent the long and short diameter of a tumor tissue respectively; RTV = $V_t/V_0$, in which $V_0$ is the tumor volume measured at the beginning of administration ($d_0$), and $V_1$ is the tumor volume measured at each time; T/C (%) = $T_{RTV}/C_{RTV}$ × 100%, in which $T_{RTV}$ is the RTV for a treatment group, and $C_{RTV}$ is the RTV for the negative control group. Results are expressed as x±SD, and one-way analysis of variance was performed by using SPSS 16.0 statistical software.

② Efficacy evaluation

Efficacy of the test samples on nude-mouse transplanted tumor of human carcinoma was evaluated by T/C (%). The current national determination criterion is as the following: T/C (%) > 60% means ineffectiveness, T/C (%) ≤ 60% with $P < 0.05$ by statistical treatment means efficient. Determination may also be made by reference to Fodstad criterion: T/C (%) > 50% means (-) no activity; T/C (%) ≤ 50% means (+/-) marginal activity; T/C (%) ≤ 40% means (+) moderate activity; T/C (%) ≤ 25% means (++) high activity; and T/C (%) ≤ 10% means (+++) particularly strong activity. For the present study, the national criterion was adopted for evaluation.

(II) Results

Inhibitory effect of lyophilized powder of artemether on the growth of nude-mouse transplanted tumor of human gastric cancer is shown in Table 11 and Figures 6-7.

Table 11 Influence of compound H on the growth of nude-mouse transplanted tumor of human gastric cancer MKN-45 on day 19 after administration

| group | dosage (mg/kg) | mode of administ ration | number of animals | | body weight (g) | | TV ($mm^3$) | | RTV | T/C (%) |
|---|---|---|---|---|---|---|---|---|---|---|
| | | | $d_0$ | $d_{19}$ | $d_0$ | $d_{19}$ | $d_0$ | $d_{19}$ | | |
| negative | 0.15 ml/20 g | *iv* | 7 | 7 | 17.8 3 | 19.9 0 | 97.41±2 5.76 | 2670.41±8 72.83 | 28.09±7.73 | 100 |
| lyophilized powder of artemether | 3 | *iv* | 7 | 7 | 18.4 4 | 20.8 0 | 91.70±1 7.38 | 1315.32±3 83.47 | 14.24±2.53 | 50.68 |
| | 10 | *iv* | 7 | 6 | 18.1 4 | 21.3 1 | 91.23±2 2.01 | 1255.05±5 35.19 | 13.61±4.05* | 48.43 |
| | 30 | *iv* | 7 | 7 | 18.6 7 | 17.5 1 | 91.58±1 4.71 | 1228.75±5 79.02 | 13.29±5.28* | 47.32 |
| | 10+$FeSO_4$ 3 mg/kg | *iv+ig* | 7 | 7 | 18.0 1 | 20.3 4 | 95.17±1 0.01 | 1291.73±3 01.77 | 13.59±2.93 | 48.38 |
| | 10+DFO 30 mg/kg | *iv+im* | 7 | 7 | 17.7 9 | 20.1 5 | 97.02±3 6.94 | 2371.43±5 67.84 | 25.83±5.81 | 91.95 |
| | 10+Vit C 200 mg/kg | *iv+im* | 7 | 7 | 17.5 5 | 19.9 5 | 92.14±2 0.97 | 2495.81±8 18.17 | 27.07±5.53 | 96.36 |
| positive 5-Fu | 10 mg/kg | *iv* | 7 | 7 | 17.8 0 | 17.2 7 | 95.75±2 0.63 | 1018.60±2 99.89 | 10.60±2.28* | 37.72 |

Note: iv: administered by intravenous injection; *ig*: intragastric administration; *im*: intramuscular injection; compared to the negative group, **P* < 0.05
$d_0$: time for administration in each cage; $d_n$: optimal efficacy time for actual treatment

The above results show that, upon administration of the lyophilized powder of artemether for 1~5 days, the tumor growth rate of each group has no significant difference therebetween; upon administration for 15~23 days, the tumor growth rate of the negative control group is obviously more rapid than that of other groups. On day 24 after administration, all the animals were sacrificed by cervical dislocation, and tumor tissues were taken for measurement of the volume. Each parameter was calculated.

On day 19 after intravenous administration of the positive control 5-Fu injection, the relative proliferation rate of the tumor (T/C %) is 37.72% (less than 60%), with significant difference by statistical treatment as compared to the negative control group, $P < 0.05$; on day 19 after intravenous administration of the lyophilized powder of artemether, T/C % of the tumor in the low dosage group is 50.68% (less than 60%), with no significant difference by statistical treatment as compared to the negative control group, $P > 0.05$; T/C % of the tumor in the middle and high dosage groups is 48.43% and 47.32% respectively (both less than 60%), with significant difference by statistical treatment as compared to the negative control group, $P < 0.05$; on day 19 after intravenous administration of lyophilized powder H, T/C % of the tumor in the middle dosage + iron group is 48.38%, with $P=0.05$ as compared to the negative control group; T/C % of the tumor in the middle dosage + Vit C group is 96.36%, with no significant difference by statistical treatment as compared to the negative control group, $P > 0.05$; and T/C % of the tumor in the middle dosage + DFO group is 91.95%, with no significant difference by statistical treatment as compared to the negative control group, $P> 0.05$.

IV. Results

It has been reported in a large number of literatures that, artemisinin and derivatives thereof, especially dihydroartemisinin and artesunate, have remarkable anti-tumor activity. Artemether is one of the derivatives of artemisinin, which has better antimalarial effect and a better *in vivo* process. On the basis of the manufacture of a lyophilized powder preparation of artemether, the *in vitro* and *in vivo* anti-tumor activity of artemether and the lyophilized powder preparation thereof were detected in the present study. According to the currently acknowledged mechanism of action of oxygen free radicals, different factors in *in vivo* experiments were designed to verify the mechanism of the anti-tumor effect thereof.

In this experiment, with seven human tumor cell lines as models, the *in vitro* anti-tumor activity of artemether and the lyophilized powder thereof were detected by using a modified MTT method, and compared with that of artemisinin, dihydroartemisinin and artesunate. The results show that, both artemether and the lyophilized powder thereof have no remarkable inhibitory effect on the seven human tumor cell lines *in vitro*: human leukemia cell lines (HL-60, K562), a human lung cancer cell line (A549), human colon cancer cell line (HT-29) and human prostate cancer cell line (PC-3), human gastric cancer cell line (MKN-28), human laryngeal cancer cell line (Hep2). Dihydroartemisinin has relatively remarkable inhibitory effect on five of the tumor cell lines: HL-60, K562, MKN-28, HT-29 and Hep2, especially being sensitive to MKN-28 with an $IC_{50}$ of 16.22 $\mu$M. As the lyophilized powder of artemether is a water-soluble preparation which is different from other derivatives in the *in vitro* studies, it is possible to perform *in vivo* anti-tumor assessment by intravenous injection. Therefore, although experimental results show that neither artemether nor the lyophilized powder of artemether has remarkable *in vitro* cytotoxicity, it may metabolically convert into dihydroartemisinin in an organism in consideration of retaining the pharmacophore peroxide bridge of artemisinin. As such, we conducted the study on *in vivo* anti-tumor effect by using the lyophilized powder of artemether.

The *in vivo* experimental results show that, upon administration of lyophilized powder of artemether by intraperitoneal injection at a dosage of 5, 10, 20 mg/kg once a day for continuous 10 days, the tumor inhibition rates of mouse sarcoma S180 were 29.06%, 44.96% and 54.93%, respectively, showing remarkable tumor inhibitory effect and good dosage dependence; and each dosage group has a significant difference by statistical analysis as compared with a negative control group. When comparing administrations by intraperitoneal injection and intravenous injection at a dosage of 10 mg/kg respectively, it has been found that, the effect of intravenous administration is superior to that of intraperitoneal administration, with the tumor inhibitory rates being 44.96% (ip) and 51.48% (iv), respectively.

Based on the above experiments, according to the currently acknowledged mechanism of action of oxygen free radicals, mouse liver cancer H22 was used as a model in the present invention, and the dosage and study protocol was adjusted. Different influencing factors were designed and the dosage was adjusted in *in vivo* experiments, so as to verify the mechanism of its anti-tumor action. The results showed that, administration of the lyophilized powder of artemether by intravenous injection at a dosage of 3, 10, 30 mg/kg, once a day for continuous 10 days, showing apparent tumor inhibitory effect and good dose dependence, and the tumor inhibitory rates thereof were 38.98%, 54.80% and 73.07%, respectively. On the basis of the 10 mg/kg dosage of the lyophilized powder of artemether, we separately designed an iron addition group, Vit C addition group and deferoxamine (DFO) addition group, i.e.: 10 mg/kg (iv), 10 mg/kg+$FeSO_4$ 3 mg/kg (ig), 10 mg/kg+Vit C 50 mg/kg (im) and 10 mg/kg+DFO 30 mg/kg (im). The purpose is to investigate the role of $Fe^{2+}$ in the anti-tumor effect of artemether. Compound ferrous sulfate and folic acid provides ferrous ions; deferoxamine (DFO) is an iron chelator which is capable of forming a chelate with *in vivo* ferrous ions $Fe^{2+}$ to remove the *in vivo* ferrous ions; and the Vit C group is used against the *in vivo* oxidation reaction of a mouse. The tumor inhibitory rates of the groups were 54.80%, 61.15%, 59.60% and 39.92%, respectively. Compared with administration of the

lyophilized powder of artemether alone, the tumor inhibitory rate in the iron addition group increased, while that in the DFO group decreased, which suggested that the mechanism of the anti-tumor effect of the lyophilized powder of artemether is related to $Fe^{2+}$ and closely related to $Fe^{2+}$ content. In consideration of the short experiment period, endogenous $Fe^{2+}$ inside the animal body did not have apparent change in short time and could not sufficiently display the inhibitory effect on tumor growth. Owing that a nude-mouse tumor xenograft human carcinomas model retains biological properties of human tumor to a large extent, and grows more slowly than the tumor xenograft from an animal, thus differs greatly in biological properties from animal-derived tumor and retains to a large extent the biological properties of human tumor. Therefore, we conducted experiments using a nude-mouse tumor xenograft human carcinomas model, and further objectively assess the efficacy and mechanism of action thereof.

The *in vitro* cell screening results show that human gastric cancer line MKN-45 is the most sensitive to dihydroartemisinin, and thus a nude-mouse subcutaneous transplanted human gastric cancer MKN-45 tumor model was selected to conduct experiments. According to the results of H22 mouse model, the dosage of Vit C was adjusted, which was increased from original 150 mg/kg to 200 mg/kg. The lyophilized powder of artemether was administered via intravenous injection at a dosage of 3, 10g, 30 mg/kg once a day for five days a week. On day 19 upon administration, T/C (%) of tumor tissues was 50.68%, 48.43%, and 47.32%, respectively, showing the inhibitory effect on tumor growth with a certain dosage relationship. Compared with the negative control, the dosages 10 and 30 mg/kg have significant differences by statistical analysis with $P < 0.05$. The positive control group 5-Fu has a T/C (%) of 37.72% at a dosage of 10 mg/kg, which has a significant difference by statistical analysis with $P < 0.05$. It can be deemed that the model was established under the conditions, and the experimental results are credible. On day 19 upon administration of the lyophilized powder of artemether (10 mg/kg), lyophilized powder of artemether (10 mg/kg) + $FeSO_4$ (3 mg/kg), lyophilized powder of artemether (10 mg/kg) + Vit C (200 mg/kg), lyophilized powder of artemether (10 mg/kg) + DFO (30 mg/kg), T/C (%) of the tumor tissue was 48.43%, 48.38%, 96.36% and 91.95%, respectively. Compared with administration of the lyophilized powder of artemether at 10 mg/kg alone, T/C (%) of the $FeSO_4$ group has no apparent change, suggesting that under this experiment condition, exogenous iron has no apparent influence on the inhibition effect of artemether on tumor growth. However, as the time after administration goes, $FeSO_4$ gradually exhibits enhancement of the inhibition effect of artemether on tumor growth. It is assumed that at the early stage of experiments, endogenous $Fe^{2+}$ is capable of meeting the demand of artemether to exert the anti-tumor effect, but with time on, the endogenous $Fe^{2+}$ is gradually consumed, and exogenous iron gradually plays a role in the enhancement of the anti-tumor strength of the artemether. T/C (%) of the DFO group dramatically rises up, suggesting that under this experiment condition, exhaustion of the endogenous $Fe^{2+}$ inside an animal is capable of counteracting the anti-tumor effect of artemether; T/C (%) of the Vit C group dramatically rises up, suggesting that under this experiment condition, elimination of ROS inside an animal is capable of preventing artemether from exerting the anti-tumor effect thereof.

V. Conclusions

Artemether and a lyophilized powder preparation thereof have no remarkable inhibitory effect on the growth and proliferation of human tumor cells *in vitro;* however, the lyophilized powder of artemether is capable of significantly inhibiting the growth of mouse sarcoma S180 and mouse liver cancer H22 *in vivo,* and significantly inhibiting the growth of nude-mouse transplanted tumor MKN-45 of human gastric cancer, suggesting that artemether may exert its anti-tumor effect via *in vivo* metabolic activation. The mechanism of the anti-tumor action thereof is associated with the fact that *in vivo* ferrous ions ($Fe^{2+}$) medicate the generation of reactive oxygen species (ROS) and in turn induce tumor cell injuries.

## Claims

1. A pharmaceutical composition for use in the treatment of liver cancer or gastric cancer comprising artemether and an iron agent, **characterized in that** the weight ratio of artemether to the iron agent is 1:0.05 to 1:2.

2. The pharmaceutical composition for use according to claim 1, **characterized in that** the weight ratio of artemether to the iron agent is 1:0.1 to 1:1.

3. The pharmaceutical composition for use according to any one of claim 1 or 2, **characterized in that** the pharmaceutical composition further comprises a pharmaceutically acceptable excipient.

4. The pharmaceutical composition for use according to claim 3, **characterized in that** the pharmaceutical composition is an injection or lyophilized powder for injection.

## Patentansprüche

1. Pharmazeutische Zusammensetzung zur Verwendung bei der Behandlung von Leberkrebs oder Magenkrebs, umfassend Artemether und ein Eisenmittel, **dadurch gekennzeichnet, dass** das Gewichtsverhältnis von Artemether zu dem Eisenmittel 1:0,05 bis 1:2 beträgt.

2. Pharmazeutische Zusammensetzung zur Verwendung nach Anspruch 1, **dadurch gekennzeichnet, dass** das Gewichtsverhältnis von Artemether zu dem Eisenmittel 1:0,1 bis 1:1 beträgt.

3. Pharmazeutische Zusammensetzung zur Verwendung nach einem der Ansprüche 1 oder 2, **dadurch gekennzeichnet, dass** die pharmazeutische Zusammensetzung ferner einen pharmazeutisch unbedenklichen Arzneistoffträger umfasst.

4. Pharmazeutische Zusammensetzung zur Verwendung nach Anspruch 3, **dadurch gekennzeichnet, dass** die pharmazeutische Zusammensetzung eine Injektion oder ein lyophilisiertes Pulver für eine Injektion ist.

## Revendications

1. Composition pharmaceutique destinée à être utilisée dans le traitement du cancer du foie ou du cancer gastrique comprenant de l'artéméther et un agent de fer, **caractérisée en ce que** le rapport pondéral de l'artéméther à l'agent de fer est de 1:0,05 à 1:2.

2. Composition pharmaceutique destinée à être utilisée selon la revendication 1, **caractérisée en ce que** le rapport pondéral de l'artéméther à l'agent de fer est de 1:0,1 à 1:1.

3. Composition pharmaceutique destinée à être utilisée selon l'une quelconque des revendications 1 ou 2, **caractérisée en ce que** la composition pharmaceutique comprend en outre un excipient pharmaceutiquement acceptable.

4. Composition pharmaceutique destinée à être utilisée selon la revendication 3, **caractérisée en ce que** la composition pharmaceutique est une injection ou une poudre lyophilisée pour injection.

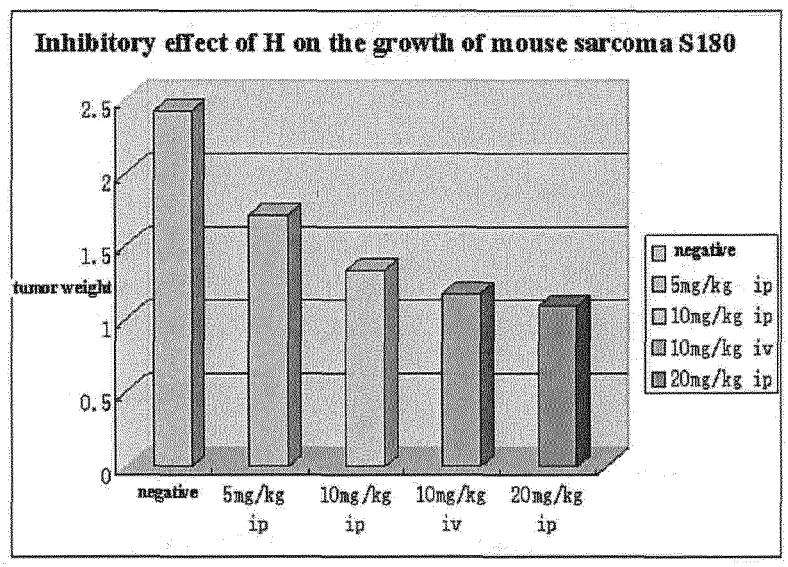
Inhibitory effect of H on the growth of mouse sarcoma S180
2.5
2
1.5
1
0.5
0
tumor weight
negative
5mg/kg ip
10mg/kg ip
10mg/kg iv
20mg/kg ip

Fig.1

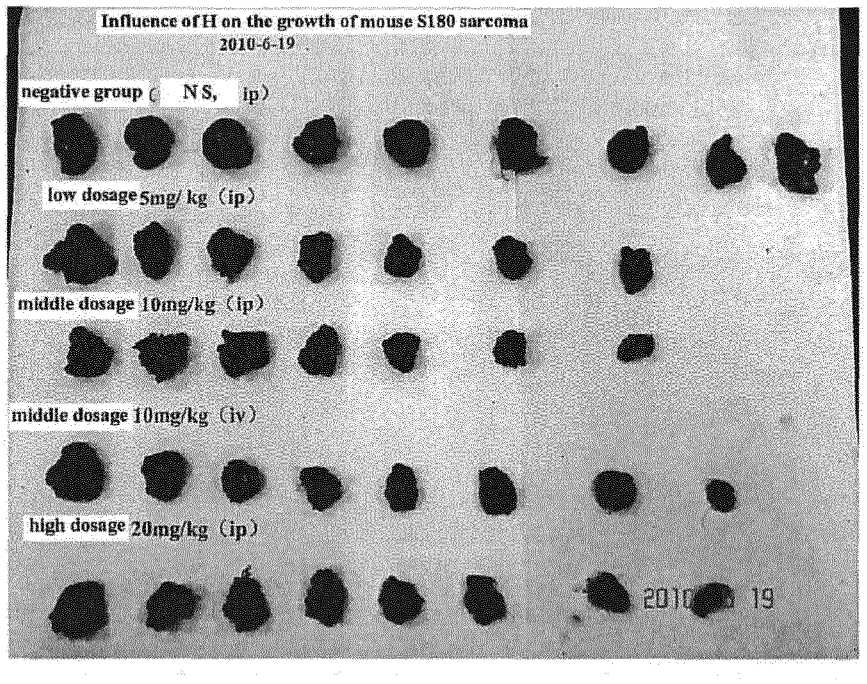
Influence of H on the growth of mouse S180 sarcoma
2010-6-19
negative group ( NS, ip)
low dosage 5mg/ kg (ip)
middle dosage 10mg/kg (ip)
middle dosage 10mg/kg (iv)
high dosage 20mg/kg (ip)

Fig. 2

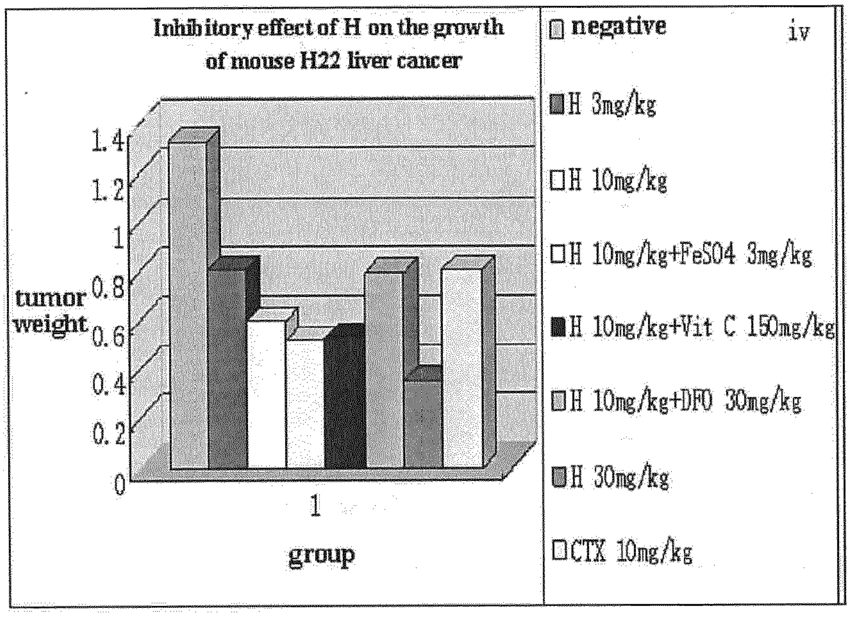
Inhibitory effect of H on the growth of mouse H22 liver cancer
negative
iv
H 3mg/kg
H 10mg/kg
H 10mg/kg+FeSO4 3mg/kg
H 10mg/kg+Vit C 150mg/kg
H 10mg/kg+DFO 30mg/kg
H 30mg/kg
CTX 10mg/kg
tumor weight
1.4
1.2
1
0.8
0.6
0.4
0.2
0
1
group

Fig. 3

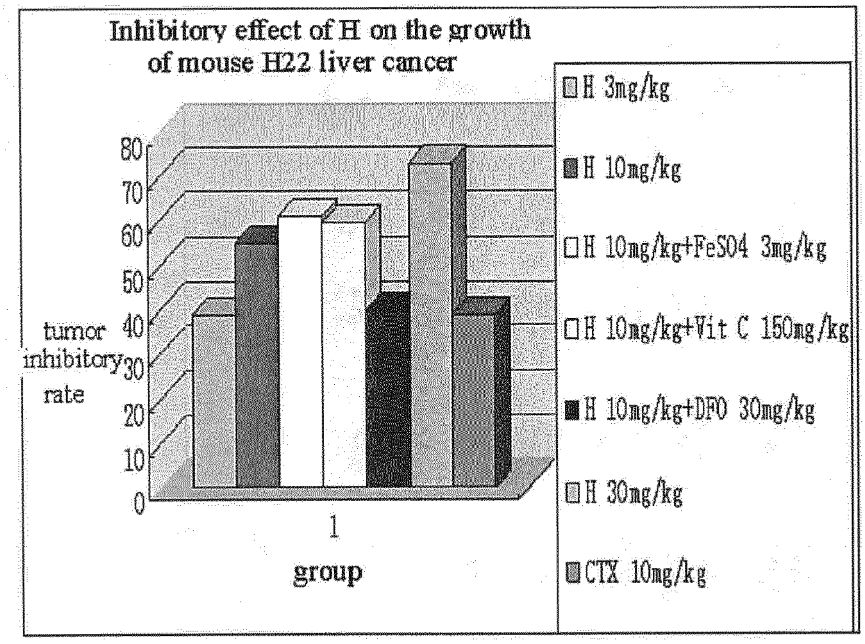
Inhibitory effect of H on the growth of mouse H22 liver cancer
H 3mg/kg
H 10mg/kg
H 10mg/kg+FeSO4 3mg/kg
H 10mg/kg+Vit C 150mg/kg
H 10mg/kg+DFO 30mg/kg
H 30mg/kg
CTX 10mg/kg
tumor inhibitory rate
80
70
60
50
40
30
20
10
0
1
group

Fig.4

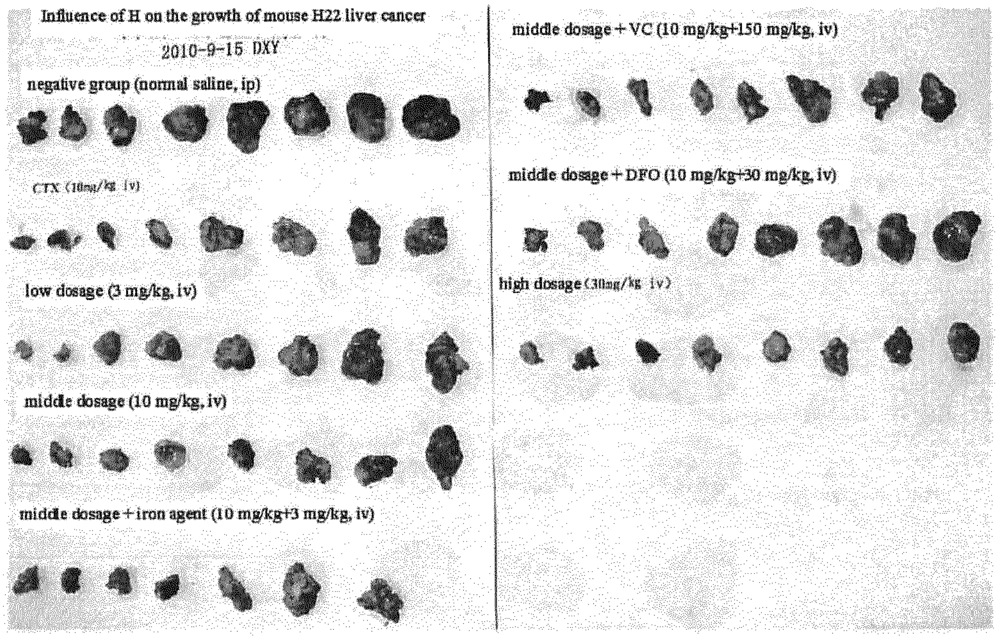
Influence of H on the growth of mouse H22 liver cancer
2010-9-15 DXY
negative group (normal saline, ip)
CTX (10mg/kg iv)
low dosage (3 mg/kg, iv)
middle dosage (10 mg/kg, iv)
middle dosage + iron agent (10 mg/kg+3 mg/kg, iv)
middle dosage + VC (10 mg/kg+150 mg/kg, iv)
middle dosage + DFO (10 mg/kg+30 mg/kg, iv)
high dosage (30mg/kg iv)

Fig.5

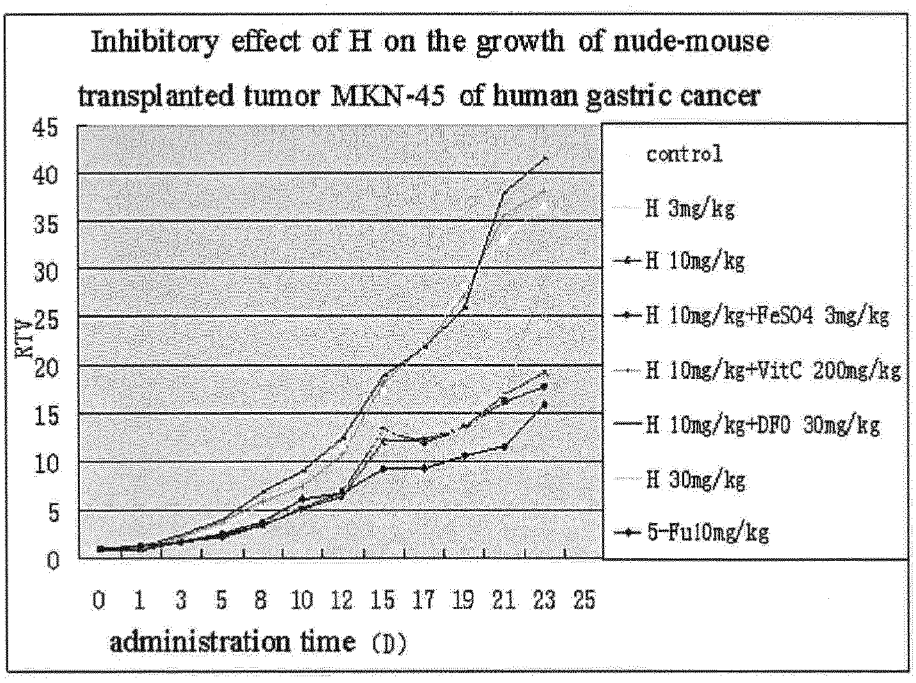
Inhibitory effect of H on the growth of nude-mouse transplanted tumor MKN-45 of human gastric cancer
RTV
45
40
35
30
25
20
15
10
5
0
0 1 3 5 8 10 12 15 17 19 21 23 25
administration time (D)
control
H 3mg/kg
H 10mg/kg
H 10mg/kg+FeSO4 3mg/kg
H 10mg/kg+VitC 200mg/kg
H 10mg/kg+DFO 30mg/kg
H 30mg/kg
5-Fu10mg/kg

Fig. 6

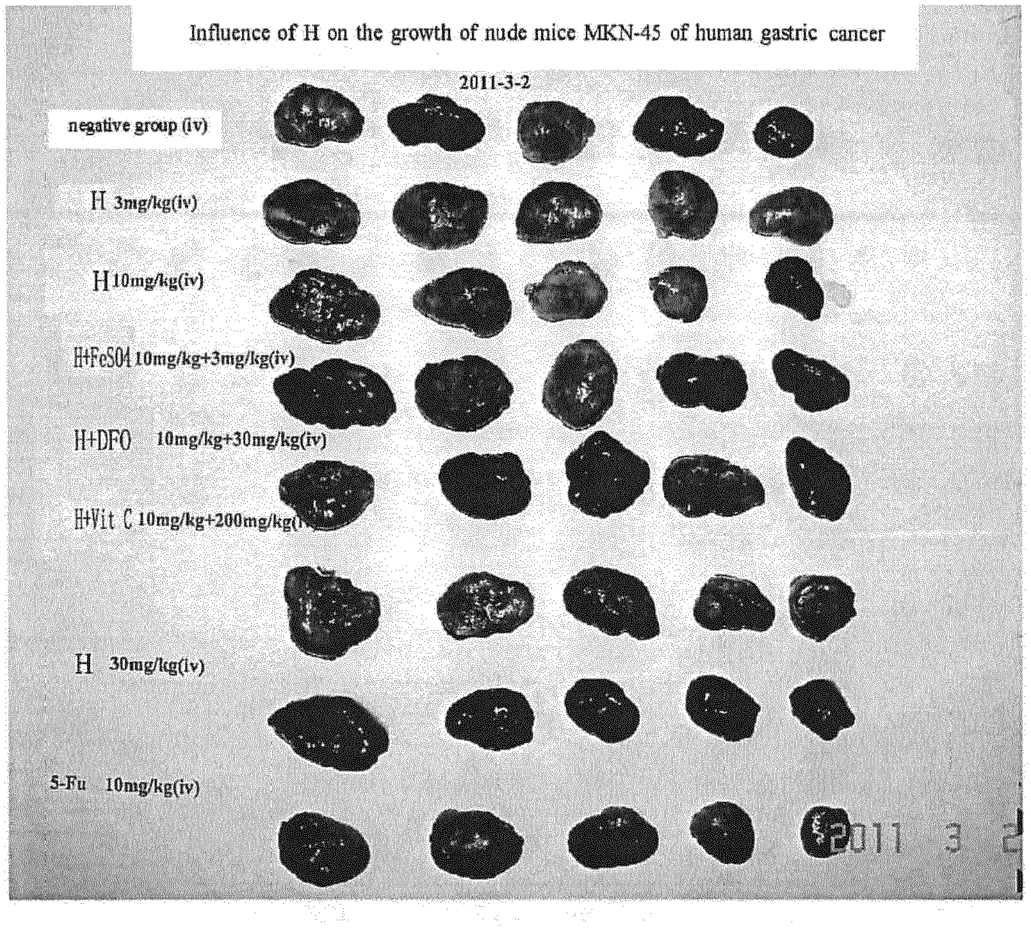
Influence of H on the growth of nude mice MKN-45 of human gastric cancer
2011-3-2
negative group (iv)
H 3mg/kg(iv)
H 10mg/kg(iv)
H+FeSO4 10mg/kg+3mg/kg(iv)
H+DFO 10mg/kg+30mg/kg(iv)
H+Vit C 10mg/kg+200mg/kg(iv)
H 30mg/kg(iv)
5-Fu 10mg/kg(iv)
2011 3 2

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 1327842